# EUROPEAN PATENT SPECIFICATION

(11) **EP 2 410 997 B1**
(45) Date of publication and mention of the grant of the patent: **21.10.2015**
(21) Application number: 10722162.4
(22) Date of filing: 23.03.2010
(51) Int. Cl.: A61K 9/20, A61K 9/28

(54) **COMPOSITION FOR THE ADMINISTRATION OF POLYMERIC DRUGS**
ZUSAMMENSETZUNG ZUR VERABREICHUNG VON POLYMER-ARZNEIMITTELN
COMPOSITION POUR L'ADMINISTRATION DE MÉDICAMENTS POLYMÈRES

(30) Priority: 23.03.2009 GB 0904942
(43) Date of publication of application: 01.02.2012
(73) Proprietor: Norwegian University of Science and Technology (NTNU), 7491 Trondheim (NO)
(72) Inventor: DRAGET, Kurt, lngar, N-7491 Trondheim (NO); TAYLOR, Catherine, N-7491 Trondheim (NO)
(74) Representative: Dzieglewska, Hanna Eva
(86) International application number: PCT/GB2010/000538
(87) International publication number: WO 2010/109180

(56) References cited:
- WO-A1-01/17506
- WO-A1-2005/089722
- WO-A2-01/72278
- WO-A2-2008/125828
- US-A1- 2005 158 392

## Description

This invention relates to improvements in and relating to pharmaceutical compositions for oral administration of polymeric biological drug substances and to methods of treatment using such compositions.

Patient acceptance of drug treatment, and the ease of drug administration, are significantly higher when the drug substance can be administered orally than when, for example, it has to be injected. Accordingly, the majority of pharmaceutical compositions supplied commercially are formulated for oral administration, e.g. as tablets, capsules or in liquid form.

Oral administration, however, is not always feasible or straightforward, for example where the drug substance is unstable in gastric fluid and so fails to reach the intestines where it may be taken up from the gut. The conventional approach to instability to gastric juice has been to administer such drug substances in tablet, capsule or dispersion form, with the tablets, capsules or particles being provided with an enteric coating, i.e. a coating of a material which is insoluble in the stomach but breaks down lower down the gut to release the drug substance it has been used to encapsulate. Enteric coating materials are well known and widely available commercially. Nevertheless, even though the provision of such release-delaying coatings is well established technology, polymeric biological drug substances, e.g. hormones and other peptides, have yet to be successfully formulated for oral administration.

There is thus a continuing need for pharmaceutical dosage forms suitable for the oral administration of polymeric biological drug substances.

We have now found that this need is addressed by the inclusion with the polymeric biological drug substance in an enteric coated oral administration form of an oligouronate, that is a linear oligosaccharide consisting majoritatively of uronate monomer residues. The invention is predicated, in part, on the surprising finding that the penetrability of intestinal mucus is dramatically increased by oligouronates. This finding has enabled the development of drug formulations for oral administration of polymeric drugs, macromolecular drugs in particular, as claimed herein which take advantage of this newly-discovered effect of oligouronates.

For certain treatments, drug substances may be administered mucosally, i.e. brought into contact with a mucosal surface of the human or animal body, e.g. a surface within the gastrointestinal surface, the airway or the vagina. In the case of fish, the mucosal surface may be the skin and vaccines may be administered mucosally to fish, such as salmon, by topical application to the skin or application into the surrounding water. The compositions of the invention are suitable for mucosal application and the invention is claimed accordingly. Oral compositions and administration however are preferred.

Thus viewed from one aspect the invention provides an oral and/or mucosal pharmaceutical composition comprising an enteric-coated drug substance, wherein said composition also comprises an enteric-coated oligouronate and wherein said drug substance is a polymeric biological drug substance.

Viewed from a further aspect the invention also provides a method of treatment of a human or non-human (e.g. avian, reptilian, piscine, or preferably mammalian) subject, which method comprises orally or mucosally administering to said subject an effective dose of a drug substance to combat a condition responsive to said drug substance, the improvement comprising administering said drug substance in the form of a composition according to the invention.

Viewed from another aspect the invention provides the use of an oligouronate for the manufacture of a pharmaceutical composition according to the invention or use in a method of treatment according to the invention.

The compositions of the invention may be in any orally or mucosally administrable form in which the polymeric biological drug substance and the oligouronate are allowed to transit the stomach and be released at the same section of the subsequent parts of the gastrointestinal tract at the same time. Thus the dosage form may be a dispersion, tablet, capsule, chewable gel, etc. Preferably, however, the composition will take the form of a tablet or capsule, coated as a single unit with the enteric, i.e. gastric-juice resistant, coating and/or containing smaller particles provided with such coatings. Preferably, the oligouronate and the polymeric biological drug substance are provided with the same coating(s) as, while not wishing to be bound by theory, it is thought that the oligouronate serves to promote bio-uptake of the polymeric biological drug substance by modifying the permeability of the mucus layer on the lumen of the gut in such a way that the polymeric drug is more able to pass through and reach the surfaces of the cells at the gut lumen. It is also postulated that the bioavailability and uptake of the polymeric drug will be affected synergistically by co-administration with a oligouronate in an enteric coated formulation. In particular, where the oligouronate and drug are released substantially simultaneously, the level of oligouronate is expected to lead to an advantageously increased bioavailability and uptake of the drug.

The material used for the enteric coating may, for example, be any of the materials conventionally used to delay release of drug substances until after the composition has passed out of the stomach. Examples include synthetic and semi-synthetic polymers, such as cellulose acetate phthalate and those available under the trade name Eudragit. The coating should be insoluble in the stomach and should prevent passage of gastric juice components, such as acids, that may break down the polymeric biological drug substance. Such coatings may be applied in conventional fashion and in conventional thicknesses/amounts. If desired, a buffer may be included with the biological drug substance, or within a coating layer, to protect the drug substance still further from any possible leakage of acid before stomach transit is complete.

The polymeric biological drug substance in the compositions of the invention may be any material which is susceptible to breakdown by gastric fluids, which is a polymer of biological origin, or is an analog or derivative of a polymer of biological origin, which has a desired physiological activity as a drug substance (rather than simply as a nutrient for example), and is not an oligosaccharide. The molecular weight of the drug substance will preferably be 500 to 500k Da, particularly 1 to 50 kDa, especially 3 to 25 kDa. Typically, the biological drug substance will be a peptide, e.g. an oligopeptide or polypeptide, for example a protein or protein fragment, and in particular a hormone. The polymer may be a derivative, e.g. a salt, ester, amide, complex or conjugate. Such derivatives are considered still to be polymeric biological drug substances as their pharmacophore, i.e. the component responsible for the desired physiological activity, remains the polymeric biological component. Particularly preferred hormones/proteins/peptides include:
insulin;
anti-tumour necrosis factor (anti-TNF);
interferons;
coagulation factors (e.g. factor VII, factor VIII and
factor IX);
follicle-stimulating hormones (FSH);
erythropoietin;
human beta-glucoserobidase; and anti-cancer agents such as :
   granulocyte-colony stimulating factor (G-CSF);
   herceptin; and
   anti-CD20.

Further preferred drug substances include antigens for oral or mucosal vaccination, e.g. protein fragments from parasitic or infective species optionally conjugated to an immunogenic carrier. Such species may for example be bacteria, viruses, yeasts or fungi. The vaccines may be used for human vaccination; however they are particularly preferred for administration to farmed animals, in particular fish and shellfish, e.g. salmon, trout, cod and prawn.

Most of these polymeric biological drug substances are available commercially, either generically or under trade names such as: Enbrel, Remicade, Humira, Avonex, Rebif, Betaseron, Pegasys, Procrit, Epogen, Reconorm, Epogin, Epomax, Eprex, Trazumab, Rituxan, Neupogen, Neulastra, and Cerezyme. The conditions they are used to treat cover a wide range, e.g. diabetes, cancer, cardiovascular disease, infertility, and Gaucher's disease.

The dosage of the polymeric biological in the compositions of the invention will typically be in the range of 5 to 100%, especially 10 to 60%, more particularly 20 to 50% of the desired daily dose per dosage unit (or per prescribed dosage volume for a composition not in dosage unit form, e.g. a liquid dispersion). The desired daily dose will generally be from 1 to 10 times, for example 2 to 6 times, the daily dose taken by injection, i.e. the dosages readily determinable from current product data sheets. The ratio between known injection dosage and oral dosage according to the invention may be determined by conventional experimentation, e.g. following determination of the percentage uptake of a labelled analog taken orally and by injection in an animal model. In general, the oral dose may be expected to be 500 to 2,000%, e.g. about 1,000%, of the dose normally given by injection (i.e. a dose 5 to 20-fold, especially about 10-fold the normal injection dose). The particular dosage of course will also depend, as with known administration by injection, on the species and size of the recipient, on the nature and severity of the condition being treated, and on the specific biological drug substance itself.

The oligouronate, as mentioned above, is preferably coated with a release-delaying coating in the same way as, or, more preferably, together with, the polymeric biological drug substance. This is in order to ensure that both the oligouronate and the polymeric biological drug substance are released at substantially the same place and time. Since the function of the oligouronate is thought to be largely to modify the mucosal surface of the gastrointestinal tract, the dose will preferably be higher, in mole terms, than that of the polymeric biological drug substance but need not be a function of the drug substance dose. The oligouronate dose will preferably be 10 to 1,200 mg, especially 50 to 1,000 mg, particularly 100 to 750 mg, per dose unit. While pre-treatment of the gastrointestinal tract with the oligouronate, i.e. sequential administration of oligouronate followed by polymeric biological drug substance, might be thought to be equivalent to simultaneous administration, it is thought that simultaneous administration is preferable since the potentiated uptake is then more closely limited to the desired polymeric biological drug substance.

The counter-ions for the oligouronate may be any of the physiologically tolerable ions commonly used for charged drug substances, e.g. sodium, potassium, ammonium, chloride, mesylate, meglumine, etc. Ions which promote alginate gelation, e.g. group 2 metals, however will preferably not be used. Such group 2 ions will desirably also be essentially absent from the other components of the compositions of the invention.

While the oligouronate, which is linear, may be a synthetic material, it is preferably a derivative, having a weight average molecular weight of less than 100,000 Da, of a naturally occurring polysaccharide. It is preferably a 3- to 28-mer, in particular a 4- to 25-mer, especially a 6- to 22-mer, in particular an 8- to 15-mer, especially a 10-mer, e.g. having a molecular weight in the range 350 to 6,000 Da especially 750 to 4,500 Da. It may be a single compound or it may be a mixture of oligouronates, e.g. of a range of degrees of polymerization. Moreover, the monomeric residues in the oligouronate, i.e. the monosaccharide groups, may be the same or different.

Oligouronates are readily accessible from natural sources since many natural polysaccharides contain uronic acid residues such as guluronic and galacturonic acid residues.

Polysaccharide to oligosaccharide cleavage to produce oligouronates useable according to the present invention may be performed using conventional polysaccharide lysis techniques such as enzymatic digestion and acid hydrolysis. Oligouronates may then be separated from the polysaccharide breakdown products chromatographically using an ion exchange resin or by fractionated precipitation or solubilization.

Examples of polysaccharides containing uronates include naturally occurring polysaccharides (such as xanthan, pectin, alginates, hyaluronan, heparin and chondroitin sulphate) and chemically modified polysaccharides, including but not limited to polysaccharides modified to add charged groups (such as carboxylated or carboxymethylated glycans), and polysaccharides modified to alter flexibility (e.g. by periodate oxidation). Suitable polysaccharides are discussed for example in "Handbook of Hydrocolloids", Ed. Phillips and Williams, CRC, Boca Raton, Florida, USA, 2000. The use of alginates however is especially preferred as these naturally occur as block copolymers of manuronic (M) and guluronic (G) acids and G-block oligomers can readily be produced from alginate source materials. Indeed, in general the oligouronate is preferably an oligoguluronic acid, or less preferably an oligogalacturonic acid.

Where alginates are used as the starting material for preparation of the oligouronate, the guluronic acid content may if desired be increased by epimerization with mannouronan C-5 epimerases from *A*. *vinelandii.*

Oligoguluronic acids suitable for use according to the invention may conveniently be produced by acid hydrolysis of alginic acid from *Laminaria hyperborea,* dissolution at neutral pH, addition of mineral acid to reduce the pH to 3.4 to precipitate the oligoguluronic acid, washing with weak acid, resuspension at neutral pH and freeze drying.

The use of oligouronates of the type described in WO2008/125828, the contents of which are hereby incorporated by reference, is especially preferred.

The composition of the invention may be produced and administered in conventional fashion. Besides the enteric coating material, the oligouronate and the polymeric biological drug substance, the compositions may contain other conventional pharmaceutical carriers and excipients, e.g. solvents, diluents, buffers, viscosity modifiers, colours, antioxidants, etc.

Particularly where the compositions are for administration to farmed animals, they may be in the form of a feed composition containing the enteric-coated substances, e.g. feed pellets. Such compositions may be prepared by including the enteric-coated substances with the other components of the feed or the enteric-coated substances may be absorbed into pre-prepared feed. This may be done for example in the manner disclosed in WO02/28199 using a dispersion of enteric-coated particles in the water used to soak the feed pellets.

Where antigens are to be administered mucosally, the inclusion of an enteric coating may if desired be dispensed with; nonetheless, such treatment and compositions suitable for use therefor form aspects of the invention. Thus, viewed from one aspect the invention provides a mucosal vaccine composition comprising an antigen, especially a peptidic antigen, and a physiologically tolerable oligouronate, optionally together with a tolerable carrier or excipient. Viewed from a further aspect the invention provides a mucosal vaccine kit comprising an antigen, and, enclosed separately, an oligouronate. Viewed from a still further aspect the invention provides a method of mucosal vaccination of an animal, especially a fish, which method comprises exposing a mucosal surface of said animal simultaneously or sequentially to an effective amount of an antigen, especially a water-soluble antigen, and an effective amount of an oligouronate.

The invention will now be described further with reference to the following non-limiting Figures and Examples, in which:
Figures 1A-1C show cellular uptake of microbeads in mucus-secreting cells;
Figures 2A-2B show the effects of oligouronate on transfection of HEK and HeLa cells;
Figures 3A-3C show uptake of labelled transferrin into MDCK cells;
Figures 4A-4C show uptake of labelled transferrin into MDCK cells treated with oligouronate;
Figures 5A-5C show uptake of labelled transferrin into MDCK cells treated with oligouronate which is subsequently washed off;
Figure 6 shows uptake of labelled transferrin into HeLa cells with and without oligouronate treatment;
Figure 7 shows diffusion of 0.5µm microbeads into mucus after photobleaching;
Figure 8 shows diffusion of 0.1µm microbeads into mucus after photobleaching;
Figure 9 shows diffusion of 0.2µm microbeads into mucus after photobleaching; and
Figures 10-13 are scanning electron micrographs of pig gastric mucin at 20 and 25 mg/ml concentrations, with and without oligouronate treatment.

### Example 1

### Insulin tablets

The following are mixed thoroughly and pressed to form tablet cores:

| | |
|---|---|
| Talc | 350mg/tablet |
| Magnesium stearate | 350mg/tablet |
| Insulin* | 100 Units |
| Sodium guluronate** | 300mg/tablet |

| | |
|---|---|
| * available from Alfa Chem, Kings Point, NY, US. ** G-block polymer, DP10, prepared as described in WO2008/125828. | |

The tablets cores are coated in conventional fashion with an enteric coating agent, e.g. Eudragit ® FS30D, available from Evonik Industries AG, Essen, Germany.

### Example 2

### Microbead uptake in cells with mucus layer

The ability of mucus-secreting HT29-MTX cells to take up microbeads was assessed. Cells having a discontinuous mucus layer and cells having a continuous mucus layer were investigated as follows.

HT29-MTX cells (*Clin. Otolaryngol. Allied Sci.* (2003) 28(**1**):p.39-42) were grown to confluence in 24 well plates. Dulbecco's Modified Eagle Medium, DMEM (GIBCO) was used. For wells designated confluent, mucus layer cells were grown under 3µm pore size Transwell™ filters (Corning) and all medium changes were accomplished through the filter membrane to protect the underlying mucus layer.

Growth medium was removed and replaced with 750µl of fresh medium and 250µl of either an oligouronate (G-block having a degree of polymerisation DP=20) or saline (control).

40µl of Microbeads (FluoSpheres^{®} carboxylate-modified microspheres, 0.02 µm, yellow-green fluorescent; Invitrogen) were added to test wells as a 0.02% suspension.

Incubation was performed at 37ºC for 2 hours and stopped by washing cells (x2) in cold PBS (2ml). Cold trypsin/EDTA used to detach cells (2ml), medium was added (2ml) and the cells were spun down. Cells were washed (x2) in cold PBS (2ml) and suspended in PBS (0.5ml).

Flow cytometry was performed with fluorophore excitation using the 488nm line of an argon laser with detectors optimised for the fluorphore.

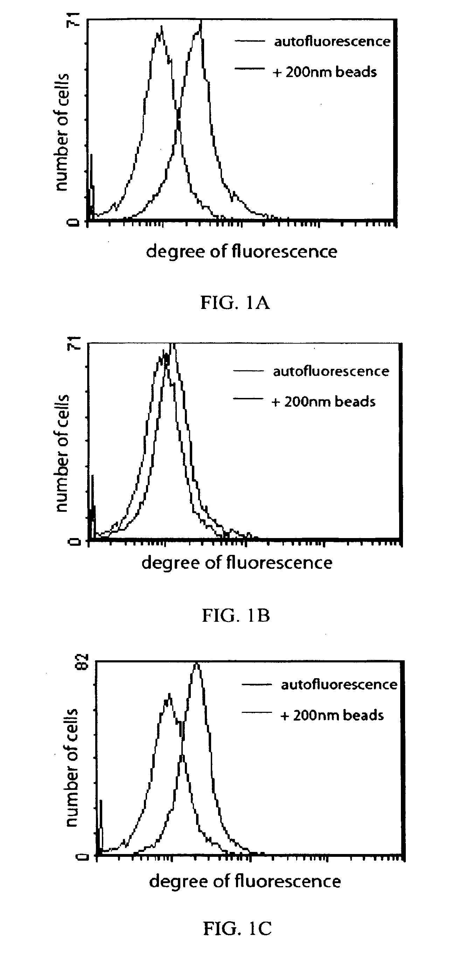

Results of this experiment are shown in Figures 1A, 1B and 1C. Figures 1A and 1B show that a continuous mucus layer is a barrier to cellular uptake of microbeads. Figure 1C shows that addition of an oligouronate significantly increases uptake of microbeads in cells with a continuous mucus layer.

### Example 3

### Effect of oligouronate on cellular uptake of a siRNA lipoplex

HeLa or HEK cells (commercially available) were grown to confluence in 6 well plates using optiMEM^{®} growth medium (Invitrogen).

The growth medium was removed and replaced with 750µl of fresh medium and 250µl of either an oligouronate (G-block having a degree of polymerisation DP=20) or saline (control).

Fluorescent SiRNA/lipofectamin™ RNAimax lipoplexes (Invitrogen) were then added to test wells according to the manufacturers recommended protocol and incubated at 37ºC for 2 hours. No transfection reagent was added to control wells.

Incubation was stopped by washing cells (x2) in cold PBS (2ml). Cold trypsin/EDTA was used to detach cells (2ml), medium was added (2ml) and the cells were spun down and washed (x2) in cold PBS (2ml). Cells were then suspended in PBS (0.5ml).

Flow cytometry was performed with fluorophore excitation using the 488nm line of an argon laser with detectors optimised for the fluorphore.

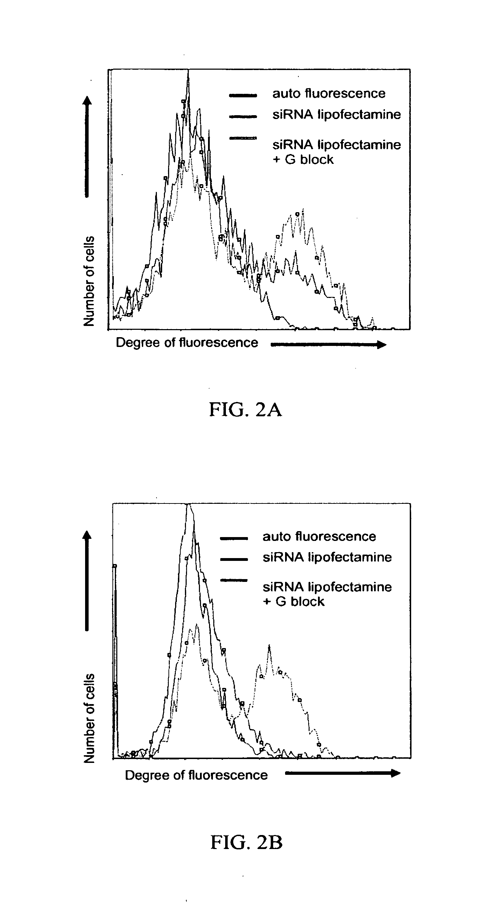

Results of this experiment are shown in Figures 2A and 2B. Figure 2A shows the effect of oligouronate on transfection of HEK cells - some uptake of nucleic acid is seen without oligouronate, but greater uptake is observed when oligouronate is present. Figure 2B shows the effect of oligouronate on transfection of HeLa cells - no uptake of nucleic acids is observed in the absence of oligouronate, however significant uptake is observed when the oligouronate is present.

### Example 4

Effect of oligouronate on cellular uptake of transferrin MDCK or HeLa cells (commercially available) were grown to confluence in 6-well plates using optiMEM^{®} growth medium (Invitrogen).

The growth medium was removed and replaced with 750µl of fresh medium and 250µl of either an oligouronate (G-block having a degree of polymerisation DP=20) or saline (control).

Cells were pre-incubated at 37ºC for 2 hours and then wells were washed (MDCK cells for washed samples only) twice with PBS.

5µg or 10µg of Alexa Fluor^{®} 488-labelled transferrin (Invitrogen) was added to test wells. Wells without transferrin were used as auto fluorescence controls. The cells were then incubated at 37ºC for 2 hours.

The incubation was stopped by washing cells (x2) in cold PBS (2ml). Cold trypsin/EDTA was used to detach cells (2ml), medium was added (2ml) and the cells were spun down. Cells were then washed (x2) in cold PBS (2ml) and suspended in PBS (0.5ml).

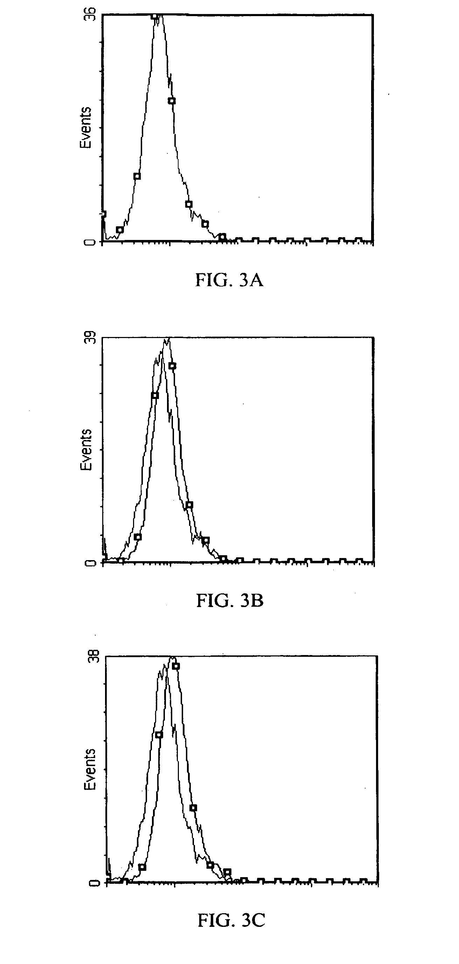

Flow cytometry was performed with fluorophore excitation using the 488nm line of an argon laser with detectors optimised for the fluorphore. Results are shown in Figures 3-6.

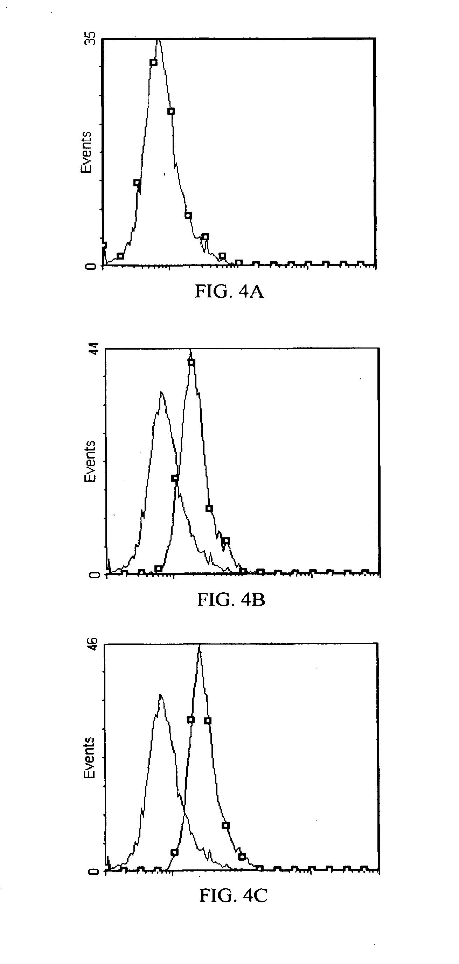

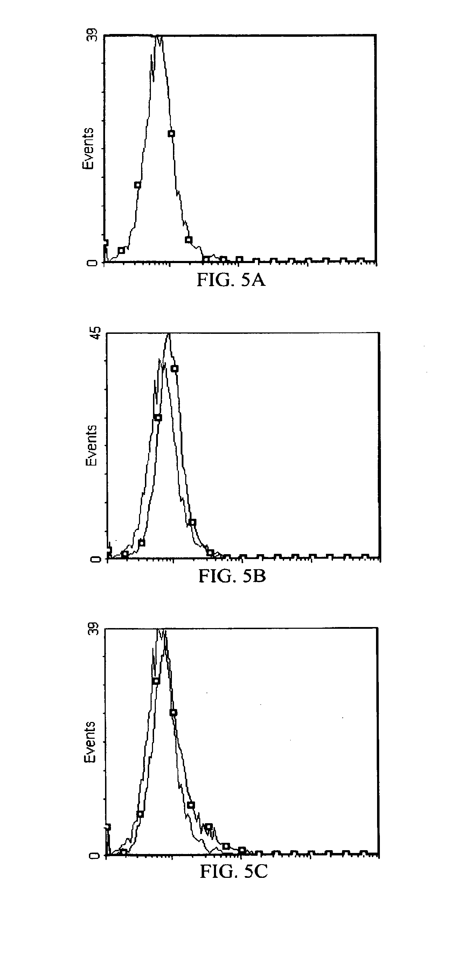

Figures 3A-3C show the results of flow cytometry of MDCK cells treated with the saline control (i.e. without oligouronate). Figures 4A-4C show the results of flow cytometry of MDCK cells treated with oligouronate. Figures 5A-5C show the results of flow cytometry of MDCK cells treated with oligouronate with the washing step included as described above. In each case, Figure A is the non-transferrin control curve; Figure B is an overlay of the control curve and the 5µg transferrin sample curve; and Figure C is an overlay of the control curve and the 10µg transferrin sample curve.

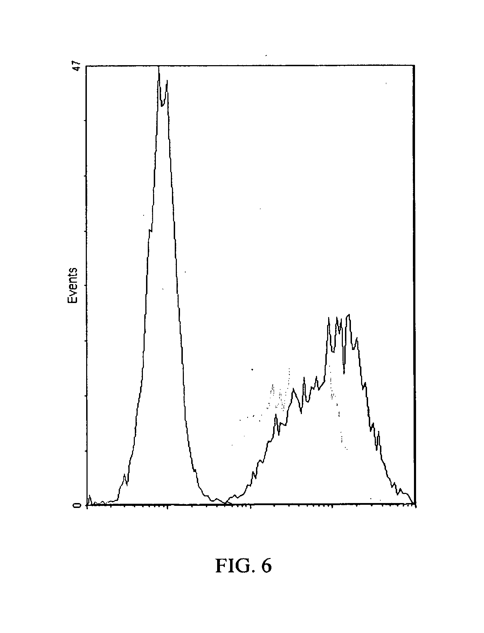

Figure 6 shows the results of flow cytometry of HeLa cells treated. This figure is an overlay of the non-transferrin control curve (left-hand peak), the 5µg transferrin sample curve without oligouronate treatment (light grey central peak), and the 5µg transferrin sample curve with oligouronate treatment (dark grey right-hand peak).

From these data it can clearly be seen that treatment with oligouronate improved uptake of tranferrin (Figs. 4A, 4B and 6). Concurrent administration improved uptake (Figs. 4), but pre-treatment with oligouronate followed by washing of cells did not increase uptake of subsequently-administered transferrin (Figs. 5).

### Example 5

### Mobility of microbeads in small intestinal mucus

Pig small intestinal mucus was scraped from mucosa of recently slaughtered pigs and frozen until use. Before use, the frozen mucus was defrosted over 24 hours at 4ºC.

The microbeads used were FluoSpheres^{®} carboxylate-modified, yellow-green fluorescent microspheres (Invitrogen) of 0.1, 0.2 and 0.5µm diameter.

### 5.1 Experiments using 0.1 and 0.5µm diameter microbeads:

Control samples were prepared by adding 32µl 0.05M NaCl solution to 260µg small intestinal mucus (prepared as described above), stirred well for 1 hour and allowed to equilibrate for 1 hour. 8µl microbeads (2% suspension) were vortexed and then added to the mucus preparation. The mixture was stirred well for 1 hour and allowed to equilibrate overnight at 4ºC.

Samples including oligouronate were prepared as for the control sample, except that 32µl 40 mg/ml oligouronate (G-block having a degree of polymerisation DP=20) in 0.05M NaCl was used in place of the 0.05M NaCl solution.

Samples were used to fill confocal imaging chambers.

A region of interest was then bleached using the 488nm line of an argon laser at full power and fluorescence recovery after photobleaching (FRAP) by diffusion was monitored at 2% laser power

### 5.2 Experiments using 0.2µm diameter microbeads:

Mucin (prepared as described above) was solubilised in 50mM NaCl at a concentration of 25mg/ml. Oligouronate (G-block having a degree of polymerisation DP=20) was solubilised in 50mM NaCl at a concentration of 30mg/ml
- Microbeads sample (without mucin)
   16µl microbead suspension was vortexed and added to 384µl 50mM NaCl.
   Final concentration 1.8×10¹¹ beads/ml
- Mucin sample (without oligouronate)
   16µl microbead suspension was vortexed and added to 64µl 50mM NaCl and 320µl mucin solution.
   Final concentration 1.8×10¹¹ beads/ml, 20mg/ml mucin
- Mucin and oligouronate sample
   16µl microbead suspension was vortexed and added to 64µl oligouronate solution. 320µl mucin solution was then added.
   Final concentration 1.8×10¹¹ beads/ml, 20mg/ml mucin, 4.8mg/ml oligouronate.

Samples were used to fill confocal imaging chambers.

A region of interest was then bleached using the 488nm line of an argon laser at full power and FRAP by diffusion was monitored at 5% laser power

### 5.3 Results:

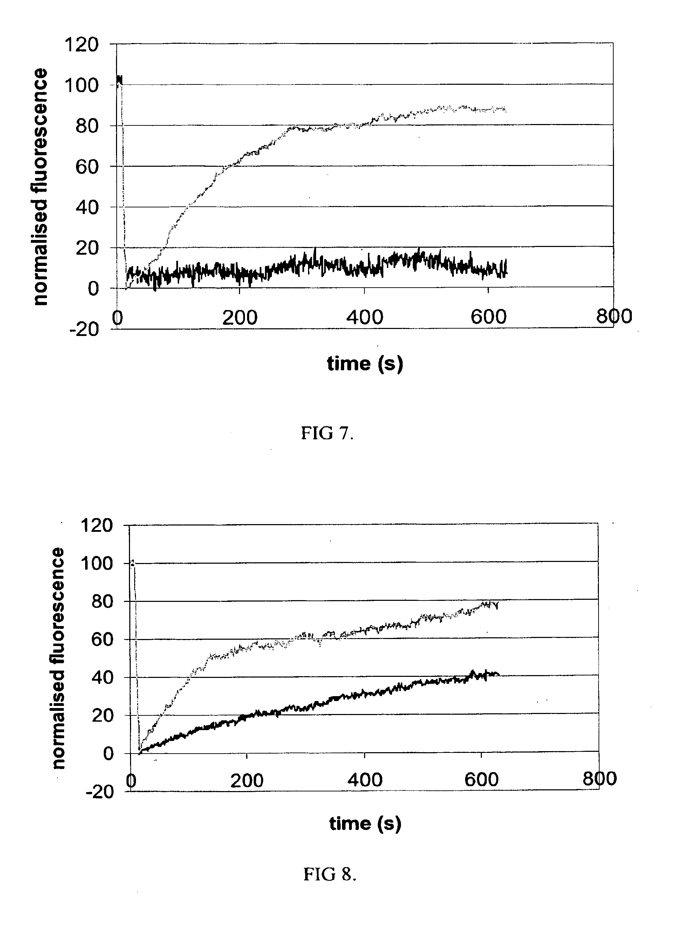

### Results are shown in Figures 7, 8 and 9.

Figure 7 shows the FRAP of 0.5µm microbeads in small intestinal mucus. The black lower line is the control sample (without oligouronate) and the grey upper line shows recovery of fluorescence in the sample treated with oligouronate.

Figure 8 shows the FRAP of 0.1µm microbeads in small intestinal mucus. The black lower line is the control sample (without oligouronate) and the grey upper line shows recovery of fluorescence in the sample treated with oligouronate.

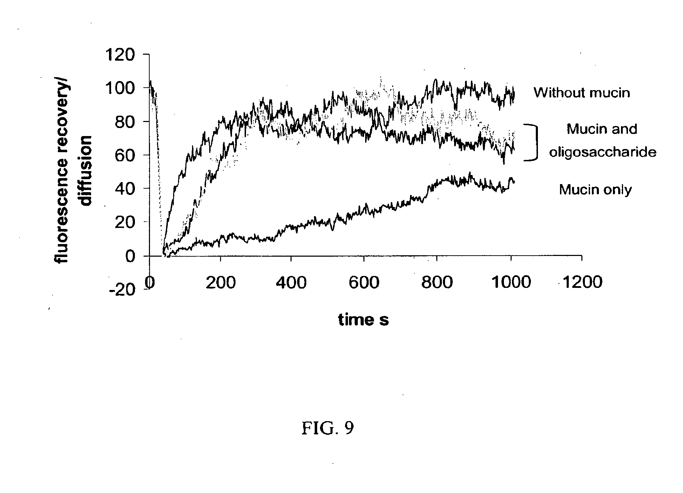

Figure 9 shows the FRAP of 0.2µm microbeads in small intestinal mucus. The black lower line is the control sample (without oligouronate), the dark grey upper line shows FRAP of the sample without mucin, and the two inner lines (dark and light) show recovery of fluorescence in mucin samples treated with oligouronate.

These data indicate that small intestinal mucus is a significant barrier to the diffusion of particles having sub-micron dimensions. Addition of oligouronate significantly reduces the diffusion barrier posed by mucus.

### Example 6

### Scanning Electron Microscopy (SEM) of gastric mucin

Mucin (pig gastric mucin prepared as described above) was solubilised in 50mM NaCl at a concentration of 25mg/ml and 30 mg/ml.

Oligouronate (as described in Example 5) was solubilised in 50mM NaCl at a concentration of 30mg/ml.

SEM samples were prepared as follows:
- Mucin (25mg/ml)
   336µl 30 mg/ml mucin was added to 64µl 0.05M NaCl and mixed.
- Mucin (20mg/ml)
   320µl 25 mg/ml mucin was added to 80µl 0.05M NaCl and mixed.
- Mucin (25mg/ml) + oligouronate
   336µl 30 mg/ml mucin added to 64µl oligouronate in 0.05M NaCl and mixed.
- Mucin (20mg/ml) + oligouronate
   320µl 30 mg/ml mucin was added to 64µl G-block in 0.05M NaCl and 16µl 0.05M NaCl and mixed.

Samples were dehydrated in graduated acetone/water, dried using critical point drying and visualised by scanning electron microscopy.

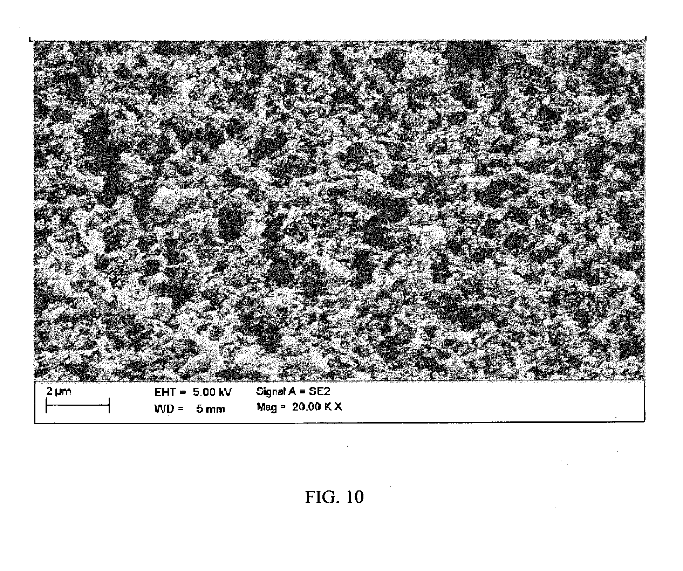

### Results are shown in Figures 10-13.

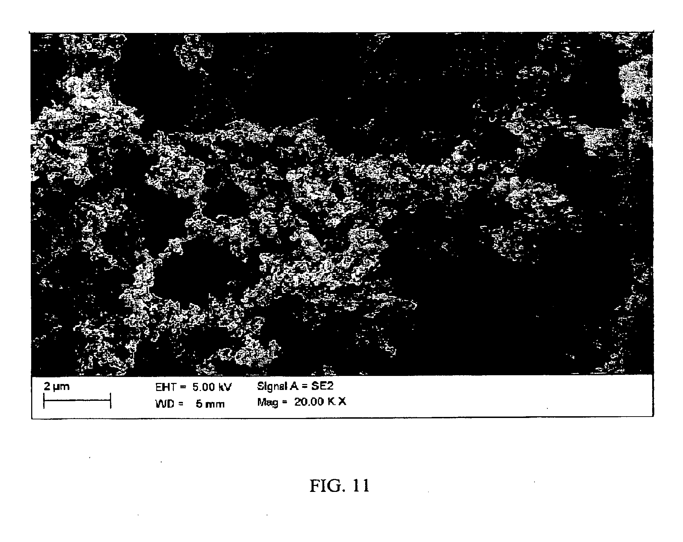

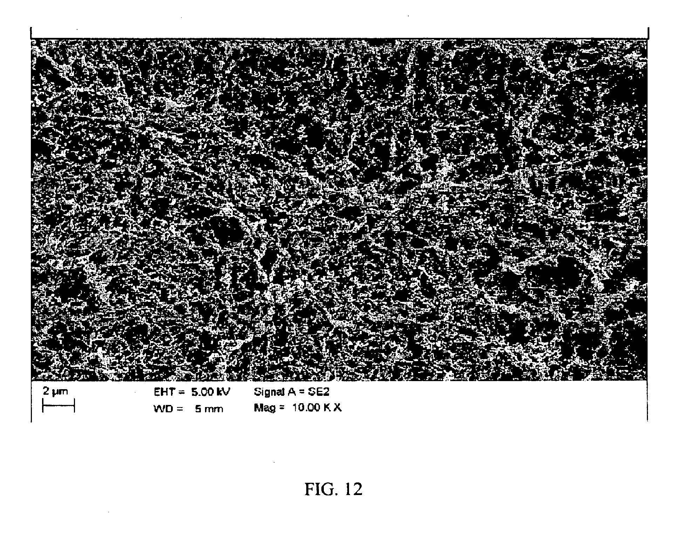

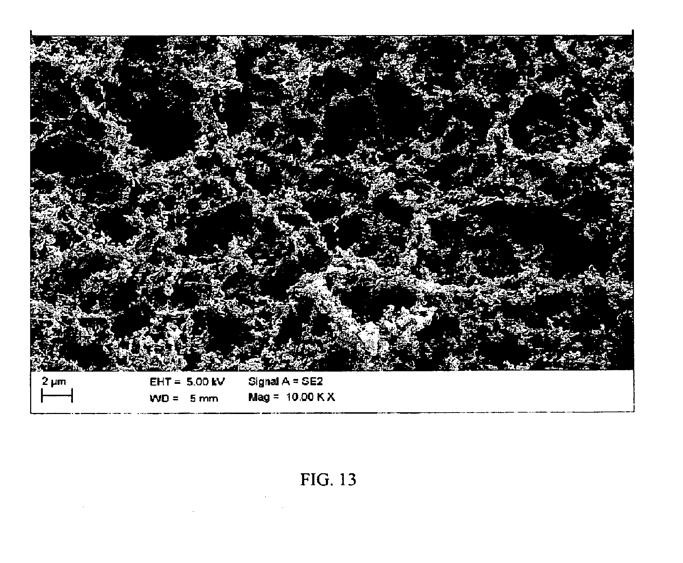

Figures 10 and 11 show the structure of the 20mg/ml mucin samples without and with 4.8mg/ml oligouronate, respectively. Figures 12 and 13 show the structure of the 25mg/ml mucin samples without and with 4.8mg/ml oligouronate, respectively.

These data indicate that addition of oligouronate to gastric mucus results in an opening of the network structure of the mucin matrix and an increase in pore size.

## Claims

1. An oral pharmaceutical composition comprising an enteric-coated drug substance, wherein said composition also comprises an enteric-coated oligouronate and wherein said drug substance is a polymeric biological drug substance.

2. A composition as claimed in claim 1 wherein said drug substance is a peptide.

3. A composition as claimed in claim 1 or 2 wherein said drug substance is insulin.

4. A composition as claimed in either of claims 1 and 2 wherein said drug substance is an antigen.

5. A composition as claimed in any one of claims 1 to 4 in coated tablet or capsule form.

6. A composition according to any one of the preceding claims for use in a method of treatment of a human or non-human subject, which method comprises orally administering to said subject an effective dose of a drug substance to combat a condition responsive to said drug substance, wherein said drug substance is provided in the form of said composition.

7. The use of an oligouronate for the manufacture of a pharmaceutical composition according to any one of claims 1 to 5 for use in a method of treatment as defined in claim 6.

8. An enteric-coated drug substance as defined in any one of claims 1 to 4 for use in a method of treatment of a human or non-human subject, which method comprises orally administering to said subject an effective dose of said drug substance to combat a condition responsive thereto, wherein the drug substance is for simultaneous administration with an enteric-coated oligouronate and wherein said drug substance is a polymeric biological drug substance.

9. A composition according to any one of claims 1 to 5 for use in therapy.

## Patentansprüche

1. Orale, pharmazeutische Zusammensetzung einer magensaftresistenten Arzneimittelsubstanz, wobei die Zusammensetzung auch ein magensaftresistentes Oligouronat umfasst und wobei die Arzneimittelsubstanz eine biologische Polymerarzneimittelsubstanz ist.

2. Zusammensetzung nach Anspruch 1, wobei die Arzneimittelsubstanz ein Peptid ist.

3. Zusammensetzung nach Anspruch 1 oder 2, wobei die Arzneimittelsubstanz ein Insulin ist.

4. Zusammensetzung nach einem der Ansprüche 1 oder 2, wobei die Arzneimittelsubstanz ein Antigen ist.

5. Zusammensetzung nach einem der Ansprüche 1 bis 4 in einer beschichteten Tablette oder in einer Kapselform.

6. Zusammensetzung nach einem der vorhergehenden Ansprüche für eine Verwendung in einem Behandlungsverfahren eines Menschen oder eines nicht menschlichen Subjekts, wobei das Verfahren ein orales Verabreichen einer wirksamen Dosis einer Arzneimittelsubstanz an das Subjekt umfasst, um einen Zustand zu behandeln, der auf die Arzneimittelsubstanz reagiert, wobei die Arzneimittelsubstanz in der Form der Zusammensetzung bereitgestellt wird.

7. Verwendung eines Oligouronats für die Herstellung einer pharmazeutischen Zusammensetzung nach einem der Ansprüche 1 bis 5 für eine Verwendung in einem Behandlungsverfahren nach Anspruch 6.

8. Magensaftresistente Arzneimittelsubstanz nach einem der Ansprüche 1 bis 4 für eine Verwendung in einem Behandlungsverfahren eines Menschen oder eines nicht menschlichen Subjekts, wobei das Verfahren ein orales Verabreichen einer wirksamen Dosis der Arzneimittelsubstanz an das Subjekt umfasst, um einen Zustand zu behandeln, der darauf reagiert, wobei die Arzneimittelsubstanz für eine gleichzeitige Verabreichung mit einem magensaftresistenten Oligouronat ist und wobei die Arzneimittelsubstanz eine biologische Polymerarzneimittelsubstanz ist.

9. Zusammensetzung nach einem der Ansprüche 1 bis 5 für eine Verwendung in der Therapie.

## Revendications

1. Composition pharmaceutique buccale comprenant une substance médicamenteuse à délitage entérique, dans laquelle ladite composition comprend également un oligo-uronate à enrobage entérique et dans laquelle ladite substance médicamenteuse est une substance médicamenteuse biologique polymère.

2. Composition selon la revendication 1, dans laquelle ladite substance médicamenteuse est un peptide.

3. Composition selon la revendication 1 ou la revendication 2, dans laquelle ladite substance médicamenteuse est l'insuline.

4. Composition selon l'une quelconque des revendications 1 et 2, dans laquelle ladite substance médicamenteuse est un antigène.

5. Composition selon l'une quelconque des revendications 1 à 4 sous forme de comprimés ou de capsules enrobés.

6. Composition selon l'une quelconque des revendications précédentes pour utilisation dans un procédé de traitement d'un sujet humain ou non humain, ledit procédé comprenant l'administration par voie buccale audit sujet d'une dose efficace d'une substance médicamenteuse pour lutter contre un état sensible à ladite substance médicamenteuse, dans laquelle ladite substance médicamenteuse est fournie sous la forme de ladite composition.

7. Utilisation d'un oligo-uronate pour la fabrication d'une composition pharmaceutique selon l'une quelconque des revendications 1 à 5 pour utilisation dans un procédé de traitement tel que défini dans la revendication 6.

8. Substance médicamenteuse à enrobage entérique selon l'une quelconque des revendications 1 à 4 pour utilisation dans un procédé de traitement d'un sujet humain ou non humain, lequel procédé comprend l'administration par voie buccale audit sujet d'une dose efficace de ladite substance médicamenteuse pour lutter contre un état sensible à celle-ci, dans laquelle la substance médicamenteuse est destinée à une administration simultanée avec un oligo-uronate à enrobage entérique et dans laquelle ladite substance médicamenteuse est une substance médicamenteuse biologique polymère.

9. Composition selon l'une quelconque des revendications 1 à 5 pour utilisation en thérapie.
